(19) Europäisches Patentamt
European Patent Office
Office européen des brevets

(11) Veröffentlichungsnummer : **0 099 976**
**B1**

# (12) EUROPÄISCHE PATENTSCHRIFT

(45) Veröffentlichungstag der Patentschrift : **20.08.86**

(51) Int. Cl.[4] : **C 07 D229/00**

(21) Anmeldenummer : **83105419.2**

(22) Anmeldetag : **01.06.83**

**(54) Neue Isocyanato-uretdione sowie ein Verfahren zu deren Herstellung.**

(30) Priorität : **24.07.82 DE 3227779**

(43) Veröffentlichungstag der Anmeldung : **08.02.84 Patentblatt 84/06**

(45) Bekanntmachung des Hinweises auf die Patenterteilung : **20.08.86 Patentblatt 86/34**

(84) Benannte Vertragsstaaten : **BE DE FR GB IT NL SE**

(56) Entgegenhaltungen :
**EP-A- 0 045 995**
**GB-A- 1 153 815**

(73) Patentinhaber : **HÜLS AKTIENGESELLSCHAFT**
**Patentabteilung / PB 15 - Postfach 13 20**
**D-4370 Marl 1 (DE)**

(72) Erfinder : **Disteldorf, Josef, Dr.**
**Am Sengenhoff 2 a**
**D-4690 Herne 1 (DE)**
Erfinder : **Hübel, Werner Dr.**
**Birnenbruchstrasse 34**
**D-4690 Herne 1 (DE)**
Erfinder : **Wolf, Elmar, Dr.**
**Stauffenbergstrasse 7**
**D-4350 Recklinghausen (DE)**

(74) Vertreter : **Steil, Hanna, Dipl.-Chem.**
**RSP PATENTE - PB 15 Postfach 1320**
**D-4370 Marl 1 (DE)**

**EP 0 099 976 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die vorliegende Erfindung betrifft Isocyanato-uretdione der Formel

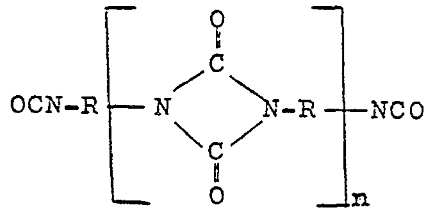

in der die einzelnen Reste R gleich oder verschieden sind und Kohlenwasserstoffreste der Formeln

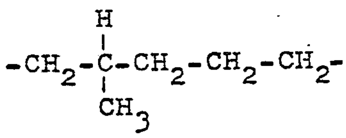

oder

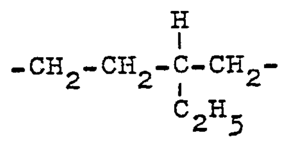

bedeuten und n eine ganze oder gebrochene Zahl von 1-5 bedeutet.

Während aromatisch substituierte Uretdione und ihre Herstellung durch Dimerisierung von aromatischen Isocyanaten mit tertiären Aminen bzw. Phosphinen als Katalysatoren bereits seit langem bekannt sind, werden erstaunlicherweise aliphatisch substituierte Uretdione dagegen erst in der DE-OS 1 670 720 zum ersten Mal beschrieben.

Die gemäß der Lehre der DE-OS 1 670 720 aus Isocyanaten hergestellten aliphatischen Uretdione enthalten allerdings in beträchtlichem Maß die entsprechenden Isocyanurate als « Verunreinigung » (Beispiel 1 : ca. 40 % Butylisocyanurat, Beispiel 2a : 49 % Ethylisocyanurat,

Beispiel 2b : 59 % Ethylisocyanurat,

Beispiel 2c : 79 % Ethylisocyanurat).

Versuche zur Dimerisierung von 2-Methyl-1.5-diisocyanato-pentan (MPDI), das gegebenenfalls bis zu 12 Gew.-% 2-Ethyl-1.4-diisocyanato-butan (EBDI) enthalten kann, entsprechend dem in der DE-OS 1 670 720 beschriebenen Verfahren führten zu einem Reaktionsgemisch mit nur maximal ca. 30 Gew.-% Uretdion.

Ein Oligomerisierungsprodukt des 2-Methyl-1.5-diisocyanato-pentans mit einem hohen Gehalt an Dimeren ist für die gezielte Weiterreaktion mit Diolen zur Herstellung von wertvollen Ausgangsverbindungen für die Polyurethanchemie von großem Interesse. Die vorliegende Erfindung zeigt ein Uretdion des 2-Methyl-1.5-diisocyanato-pentans mit einem hohen Uretdiongehalt ( > 70 %).

Gegenstand der Erfindung sind daher Isocyanato-uretdione der Formel

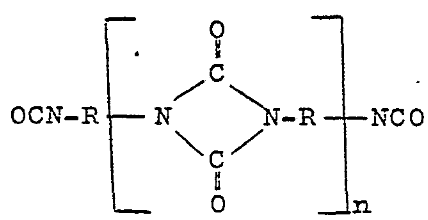

in der die einzelnen Reste R gleich oder verschieden sind und Kohlenwasserstoffreste der Formeln

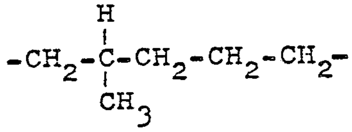

oder

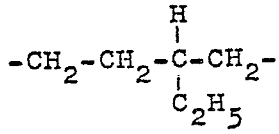

bedeuten und n eine ganze oder gebrochene Zahl von 1-5 bedeutet, die in der Hitze zu > 70 % in die Ausgangsisocyanate rückspaltbar sind.

Weiterer Gegenstand der Erfindung ist ein Verfahren zur Herstellung von Isocyanato-uretdionen gemäß angegebener Formel, welche in der Hitze zu > 70 % in die Ausgangsisocyanate rückspaltbar sind, durch katalytische Dimerisierung eines Teils der Isocyanatgruppen von aliphatischen Diisocyanaten, dadurch gekennzeichnet, daß man als aliphatische Diisocyanate ein in wesentlichen aus einem Diisocyanat der Formel

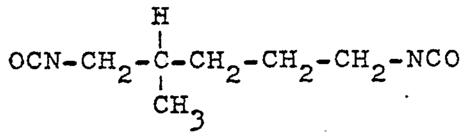

(MPDI)

und gegebenenfalls einem Diisocyanat der Formel

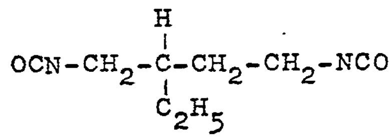

(EBDI)

bestehendes Diisocyanat bzw. Diisocyanat-Gemisch verwendet und als Katalysator eine Verbindung der allgemeinen Formel

$$X_mP(NR^1{}_2)_{3-m}$$

wobei m : 0, 1, 2
x : Cl, OR, R
$R^1$ : Methyl- oder Ethylreste
bedeuten,
bei Temperaturen von 0-80 °C, vorzugsweise 10-30 °C, einsetzt, und das Reaktionsprodukt nach einem Umsatz von 5-70, vorzugsweise 30-60 Gew.-%, ohne vorherige Desaktivierung des Katalysators aus dem Reaktionsgemisch durch Dünnschichtdestillation als Rückstand sowie Katalysator und Monomeres als Destillat isoliert.

Die Katalysatoren werden in Mengen von 0,1 bis 5 Gew.-%, vorzugsweise 0,5 bis 2 Gew.-%, eingesetzt. Besonders geeignet ist Tris-[dimethylamino]-phosphin. Die Herstellung der Phosphor-Verbindungen wird im Houben-Weyl, Bd. V, S. 108, beschrieben, deren Einsatz zur Dimerisierung von Isophorondiisocyanat, einer cycloaliphatischen Verbindung, in EP-A 45 995.

Im allgemeinen weisen die beim erfindungsgemäßen Verfahren eingesetzten Diisocyanat-Gemische folgende Zusammensetzung auf :

88 bis 99 Gew.-% 2-Methyl-1.5-diisocyanatopentan (MPDI)
12 bis 1 Gew.-% 2-Ethyl-1.4-diisocyanatobutan (EBDI)

Reines Diisocyanat der erstgenannten Formel kann anstelle der Gemische ebenfalls eingesetzt werden.

Bevorzugtes Ausgangsmaterial ist ein entsprechendes Diisocyanat-Gemisch aus ca. 88 bis 95 Gew.-% 2-Methyl-1.5-diisocyanatopentan und 12 bis 5 Gew.-% 2-Ethyl-1.4-diisocyanatobutan.

Die Herstellung der Diisocyanate bzw. Diisocyanat-Gemische erfolgt in an sich bekannter Weise durch Phosgenierung der entsprechenden Diamine (vgl. z. B. US-PS 3 631 198). Diese werden durch katalytische Hydrierung entsprechender Dinitrile erhalten, die beispielsweise als Nebenprodukt bei der Adipodinitrilherstellung durch Umsetzung von Butadien mit HCN oder bei der Dimerisierung von Acrylnitril anfallen.

Die Durchführung des erfindungsgemäßen Verfahrens erfolgt in zwei Stufen,
wobei

a) in der 1. Stufe mit Hilfe des beschriebenen Katalysators bis zu einem Umsatz von 5 bis 70 Gew.-%, vorzugsweise 30 bis 60 Gew.-%, dimerisiert wird und

b) in einem 2. Schritt das nicht umgesetzte Diisocyanat bzw. Diisocyanat-Gemisch mit dem Katalysator durch Dünnschichtdestillation vom Reaktionsprodukt abgetrennt wird.

Das abdestillierte MPDI mit gegebenenfalls EBDI (plus Katalysator) kann wieder zur Reaktion eingesetzt werden.

Die Reaktionstemperatur liegt in einem Bereich von 10 bis 80 °C, vorzugsweise 10 bis 30 °C. Die erforderliche Katalysatormenge hängt sehr von der Art des eingesetzten Katalysators ab. Mengen von 0,1 bis 5 Gew.-%, bezogen auf das eingesetzte MPDI bzw. MPDI/EBDI-Gemisch sind im allgemeinen ausreichend. Bevorzugt finden 0,5 bis 2 Gew.-% Phosphorigesäuretriamide Anwendung. Die Reaktionszeit, die Zeit in der z. B. 30 bis 60 Gew.-% MPDI (bzw. MPDI im Gemisch mit EBDI) umgesetzt sind, hängt — bei konstanter Temperatur — in hohem Maß von der Konzentration sowie von der Art des eingesetzten Katalysators ab. Sie beträgt in der Regel 3 bis 40 h. Die Reaktion kann in polaren Lösungsmitteln wie Estern, Chlorkohlenwasserstoffen, Ethern und Ketonen, oder lösungsmittelfrei durchgeführt werden. Bevorzugt arbeitet man lösungsmittelfrei.

Die Aufarbeitung des Reaktionsgemisches erfolgt, wie bereits angeführt, durch Dünnschichtdestillation bei 100 bis 120 °C und 0,013 mbar bis 0,66 mbar.

Die erfindungsgemäßen Verfahrensprodukte sind bei Raumtemperatur niedrig viskos (ca. 700 bis 1 200 mPas). Ihr NCO-Gehalt liegt im Bereich von 18 bis 23 Gew.-%, vorzugsweise 20 bis 22 Gew.-%, d. h., daß mehr oder minder hohe Anteile oligomerer Uretdione (bzw. in geringerem Maße auch Isocyanurate) des MPDI und/oder EBDI im Reaktionsprodukt vorliegen müssen.

Der Monomer-Gehalt des Reaktionsproduktes ist < 1 % ; nach dem Erhitzen auf 180 bis 200 °C (0,5 h) beträgt der NCO-Gehalt 42 bis 44 Gew.-%.

Das Uretdion des MPDI bzw. MPDI/EBDI-Gemisches soll als Zwischenprodukt zur Herstellung von Kunststoffen, Lacken und Schaumstoffen Verwendung finden. Es eignet sich, besonders in blockierter Form, vor allem mit ε-Caprolactam oder Acetonoxim als Blockierungsmittel, zur Herstellung von lösungsmittelhaltigen sowie lösungsmittelfreien Ein- und Zweikomponenten-Lacken, wie z. B. Coil Coating- und High Solid-Lacken und Polyurethan-Pulverlacken.

Beispiel 1 (Vergleichsbeispiel)

1 000 Gewichtsteile 2-Methyl-1.5-diisocyanatopentan (mit ca. 6 % 2-Ethyl-1.4-diisocyanatobutan) und 10 Gewichtsteile Tributylphosphin wurden 3 h bei Raumtemperatur stehen gelassen. In dieser Zeit sank der NCO-Gehalt von 50 % auf ca. 38 %. Ohne vorherige Desaktivierung des Katalysators wurde das Reaktionsgemisch bei 150 °C/0,133 mbar im Dünnschichtverdampfer destilliert. Der Rückstand hatte einen NCO-Gehalt von 20,5 % ; beim Erhitzen auf 180 °C (1/2 h) wurde ein NCO-Gehalt von 36,5 % gefunden.

Beispiel 2

1 000 Gewichtsteile MPDI (mit ca. 6 % EBDI) und 10 Gewichtsteile Tris-(dimethylamino)-phosphin wurden 2 h bei Raumtemperatur stehen gelassen. Nach dieser Zeit betrug der NCO-Gehalt des Reaktionsgemisches 39,3 %. Die Aufarbeitung erfolgte wie im Beispiel 1.

Der Rückstand hatte bei 25 °C eine Viskosität von 165 mPas und einen NCO-Gehalt von 20,3 % ; beim Erhitzen auf 180 °C (1/2 h) wurde ein NCO-Gehalt von 46,3 % gefunden. Das IR-Spektrum des Rückstandes ist in Fig. 1 abgebildet.

Beispiel 3

1 000 Gewichtsteile MPDI (mit ca. 6 % EBDI) und 15 Gewichtsteile Phosphorigsäure-(bisdimethylamid)-chlorid wurden bei Raumtemperatur 6 h stehen gelassen. Nach dieser Zeit betrug der NCO-Gehalt des Reaktionsgemisches 37 %. Die Aufarbeitung erfolgte wie im Beispiel 1. Der Rückstand enthielt 20,1 % NCO ; beim Erhitzen auf 180 °C (1/2 h) wurde ein NCO-Gehalt von 43,8 % gefunden.

Beispiel 4

1 000 Gewichtsteile MPDI (mit ca. 6 % EBDI) und 10 Gewichtsteile Phosphorigsäuremethylester-bisdiethylamid wurden 10 h bei Raumtemperatur stehen gelassen. Nach dieser Zeit betrugt der NCO-Gehalt des Reaktionsgemisches 36,5 %. Die Aufarbeitung erfolgte wiederum wie im Beispiel 1. Der Rückstand enthielt 20,0 % NCO ; beim Erhitzen auf 180 °C (1/2 h) wurde ein NCO-Gehalt von 44 % gefunden.

**Patentansprüche**

1. Isocyanato-uretdione der Formel

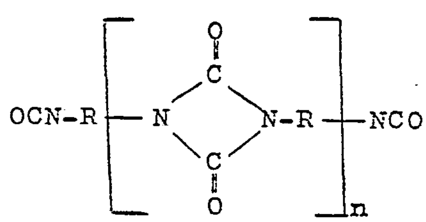

in welcher die einzelnen Rest R gleich oder verschieden sind und Kohlenwasserstoffreste der Formeln

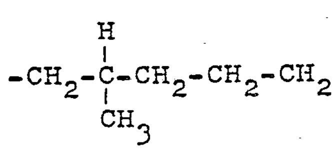

oder

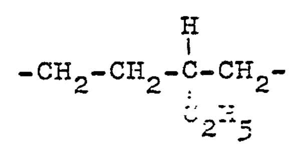

bedeuten und n eine ganze oder gebrochene Zahl von 1 bis 5 bedeutet, wobei diese Isocyanato-uretdione in der Hitze zu > 70 % in die Ausgangsisocyanate rückspaltbar sind.

2. Verfahren zur Herstellung von Isocyanato-uretdionen gemäß Anspruch 1 durch katalytische Dimerisierung eines Teils der Isocyanatgruppen von aliphatischen Diisocyanaten, dadurch gekennzeichnet, daß man als aliphatische Diisocyanate ein im wesentlichen aus einem Diisocyanat der Formel

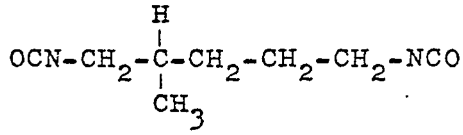

und gegebenenfalls einem Diisocyanat der Formel

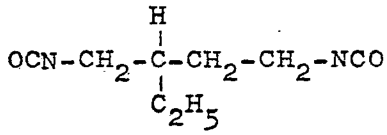

bestehendes Diisocyanat bzw. Diisocyanat-Gemisch verwendet und als Katalysator eine Verbindung der allgemeinen Formel

$$X_mP(NR_2)_3\text{—}m$$

wobei m : 0, 1, 2
X : Cl, OR, R
$R^1$ : Methyl- oder Ethylreste
bedeuten,
bei Temperaturen von 0 bis 80 °C, vorzugsweise 10 bis 30 °C, einsetzt, und das Reaktionsprodukt nach einem Umsatz von 5 bis 70, vorzugsweise 30 bis 60 Gewichtsprozent, ohne vorherige Desaktivierung des Katalysators aus dem Reaktionsgemisch durch Dünnschichtdestillation als Rückstand sowie Katalysator und Monomeres als Destillat isoliert.

3. Verfahren nach Anspruch 2, dadurch gekennzeichnet, daß man als Katalysator die Verbindung

$$P[N(CH_3)_2]_3$$

verwendet.

## Claims

1. Isocyanato-uretdiones of the formula

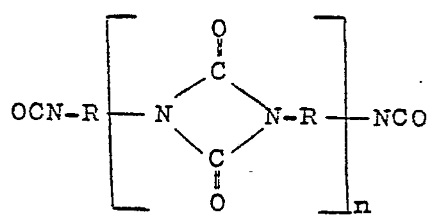

wherein the individual radicals R are identical or different and represent hydrocarbon radicals of the formulae

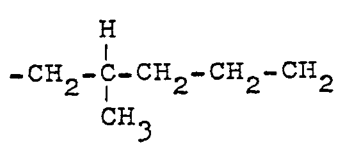 or 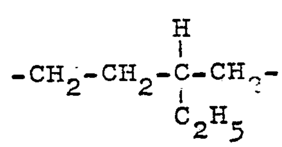

and n is an integer or a fractional number from 1 to 5, these isocyanato-uretdiones being redissociable when hot to the starting isocyanates to an extent of > 70 %.

2. Process for the preparation of isocyanato-uretdiones according to Claim 1 by catalytic dimerization of a part of the isocyanate groups of aliphatic diisocyanates, characterized in that a diisocyanate or diisocyanate mixture consisting essentially of a diisocyanate of the formula

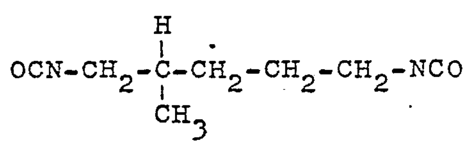

and if appropriate a diisocyanate of the formula

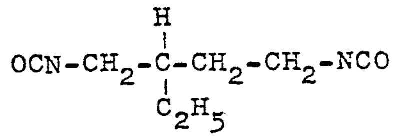

are used as the aliphatic diisocyanates and a compound of the general formula

$$X_mP(NR^1{}_2)_{3-m}$$

where m = 0, 1 or 2
X = Cl, OR or R
$R^1$ = methyl or ethyl radicals

is employed as the catalyst at temperatures of 0 to 80 °C, preferably 10 to 30 °C, and the reaction product, after a conversion of 5 to 70, preferably 30 to 60 per cent by weight, is isolated as the residue from the reaction mixture, without previous deactivation of the catalyst, by thin-film distillation, and the catalyst and monomer are isolated as the distillate.

3. Process according to Claim 2, characterized in that the compound

$$P[N(CH_3)_2]_3$$

is used as the catalyst.

**Revendications**

1. Isocyanato-uretdiones répondant à la formule :

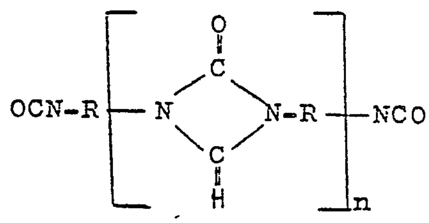

dans laquelle les différents restes R sont semblables ou différents et représentent des restes hydrocarbures de formules :

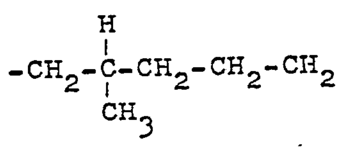 ou 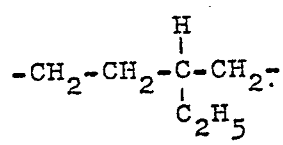

et n est un nombre entier ou fractionnel de 1 à 5, ces isocyanato-uretdiones pouvant se rescinder à la chaleur à raison de plus de 70 % dans les isocyanates de départ.

2. Procédé pour la fabrication d'isocyanato-uretdiones suivant la revendication 1, par dimérisation catalytique d'une partie des groupes isocyanates de diisocyanates aliphatiques, caractérisé en ce que l'on utilise, comme diisocyanate aliphatique, un diisocyanate, ou un mélange de diisocyanates, constitué essentiellement d'un diisocyanate répondant à la formule :

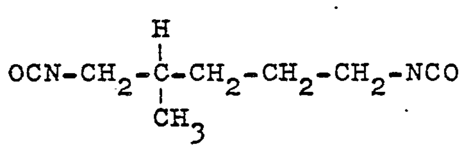

et éventuellement d'un diisocyanate répondant à la formule :

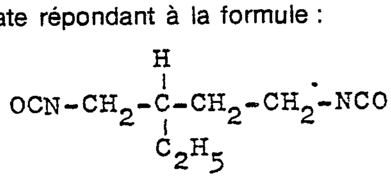

et, comme catalyseur, un composé dont la formule générale est :

$$X_mP(NR^1{}_2)_{3-m}$$

où m = 0, 1, 2
X = Cl, OR, R
$R^1$ = reste méthyl ou éthyl
à des températures de 0 à 80 °C, de préférence 10 à 30 °C, et que l'on isole du mélange réactionnel le produit de la réaction, comme résidu, ainsi que le catalyseur et le monomère, comme distillat d'une distillation en couche mince, après une conversion de 5 à 70 de préférence de 30 à 60 % en poids, sans désactivation préalable du catalyseur.

3. Procédé suivant la revendication 2, caractérisé en ce qu'on utilise comme catalyseur le composant :

$$P[N(CH_3)_2]_3.$$

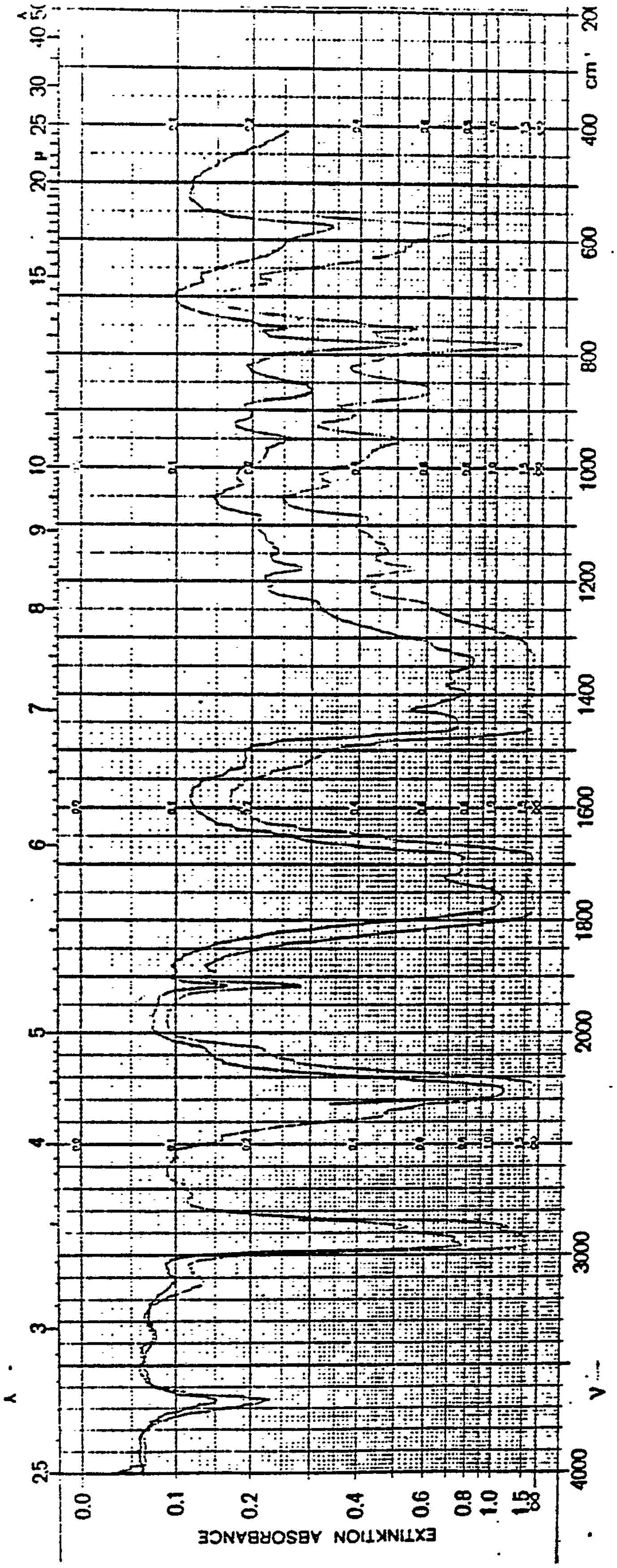


IR-Spektrum des Rückstands aus Beispiel 2 (dimeres 2-Methyl-1,5-diisocyanatopentan)